# FASCICULE DE BREVET EUROPEEN

(11) **EP 3 303 409 B1**
(45) Date de publication et mention de la délivrance du brevet: **22.05.2019**
(21) Numéro de dépôt: 16741056.2
(22) Date de dépôt: 03.06.2016
(51) Int. Cl.: C08B 30/04, A61K 31/718, C08B 30/18, C12P 19/04, C12P 19/14

(54) **PROCÉDÉ OPTIMISÉ DE DÉCONTAMINATION DE L'AMIDON UTILISÉ COMME MATIÈRE PREMIÈRE POUR L'OBTENTION DE POLYMÈRES DE GLUCOSE DESTINÉS À LA DIALYSE PÉRITONÉALE**
OPTIMIERTES VERFAHREN ZUR DEKONTAMINATION DER STÄRKE ALS ROHMATERIAL ZUR GEWINNUNG VON GLUCOSEPOLYMEREN FÜR DIE PERITONEALDIALYSE
OPTIMISED METHOD FOR DECONTAMINATING THE STARCH USED AS A RAW MATERIAL FOR OBTAINING GLUCOSE POLYMERS INTENDED FOR PERITONEAL DIALYSIS

(30) Priorité: 04.06.2015 FR 1555077
(43) Date de publication de la demande: 11.04.2018
(73) Titulaire: Roquette Frères, 62136 Lestrem (FR)
(72) Inventeur: LANOS, Pierre, 59480 La Bassee (FR); DUPONT, Thierry, 62232 Vendin Les Bethune (FR); ALLAIN, Fabrice, 59800 Lille (FR); CARPENTIER, Mathieu, 59350 Saint Andre Lez Lille (FR); HACINE-GHERBI, Héla, 59650 Villeneuve D'ascq (FR); DENYS, Agnès, 59800 Lille (FR)
(74) Mandataire: Cabinet Plasseraud
(86) Numéro de dépôt international: PCT/FR2016/051325
(87) Numéro de publication internationale: WO 2016/193634

(56) Documents cités:
- WO-A1-2010/125315
- FR-A1- 2 966 843

## Description

La présente invention est relative à l'élaboration d'un procédé optimisé de décontamination des amidons mis en oeuvre dans les circuits de production de polymères de glucose, plus particulièrement ceux destinés aux domaines médicaux, plus particulièrement encore à celui de la dialyse péritonéale.

### Arrière-plan technologique de l'invention

La société Demanderesse a choisi de développer son invention dans un domaine connu pour la dangerosité des contaminants d'origine microbienne susceptibles de se développer dans les circuits de production des polymères de glucose à l'origine de possibles réactions inflammatoires très néfastes pour la santé humaine.

Dans le cadre d'une démarche de sécurité sanitaire, il est important de s'assurer de l'absence de contaminants d'origine microbienne, tant sous forme de cellules vivantes que de débris cellulaires, par tous les moyens techniques adéquats, notamment :
- la définition de méthodes d'identification et de dosage efficaces des contaminants,
- la définition de circuits de production sécurisés, par la mise en place de dispositifs et de techniques de purification adaptés.

Dans le cas de la dialyse péritonéale, un certain nombre d'ingrédients doivent être préparés dans les conditions de pureté les plus strictes.

La dialyse péritonéale est en effet un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

Les dialysats les plus couramment utilisés sont composés d'une solution tampon (du lactate ou du bicarbonate) à pH acide (5,2 - 5,5) ou physiologique (7,4) à laquelle sont ajoutés :
- des électrolytes (sodium, calcium, magnésium, chlore) et surtout
- un agent osmotique, principalement un polymère de glucose, tel que par exemple l'« icodextrine » présent dans la solution pour dialyse péritonéale ambulatoire EXTRANEAL® commercialisée par la société BAXTER.

Dans ce domaine plus particulier de l'utilisation des polymères du glucose destinés à la dialyse péritonéale continue et ambulatoire, il est très vite apparu que ces hydrolysats d'amidon (mélange de glucose, d'oligomères et de polymères de glucose) ne pouvaient pas être utilisés tels quels.

La demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

Les procédés proposés dans la demande de brevet EP 207.676 pour obtenir ces polymères de glucose de faible indice de polymolécularité à partir d'hydrolysats d'amidon consistent :
- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Soucieuse de mettre au point un procédé de fabrication d'un polymère de glucose complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse s'est attachée à résoudre ce problème dans son brevet EP 667.356 en partant d'un amidon hydrolysé plutôt que d'une maltodextrine.

Le polymère de glucose obtenu par fractionnement chromatographique contient alors de préférence moins de 3 % de glucose et de polymères de glucose de DP inférieur ou égal à 3 et moins de 0,5 % de polymères de glucose de DP supérieur à 600.

### Les risques de contamination

Il est cependant à déplorer des risques de contamination microbienne des préparations destinées à la dialyse péritonéale.

Il est en effet connu que les circuits de production des polymères de glucose peuvent être contaminés par des microorganismes, ou par des substances pro-inflammatoires contenus dans lesdits microorganismes.

Dans le cas où le procédé de fabrication des polymères de glucose part de l'amidon, il est classiquement décrit qu'en amidonnerie, la contamination des amidons de maïs (ou de blé) est due à des microorganismes de type levures, moisissures et bactéries, et plus particulièrement par des bactéries acidothermophiles de type *Alicyclobacillus acidocaldarius* (bactéries extrémophiles qui se développent dans les zones chaudes et acides du circuit).

Le risque majeur pour le patient qui reçoit ces polymères de glucose contaminés est alors la péritonite.

Ces épisodes de péritonite sont provoqués par des infections bactériennes intrapéritonéales, et le diagnostic est habituellement facilement établi par les cultures positives de dialysat.

La « péritonite stérile », décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéale, des cas isolés de péritonite aseptique ont été rapportés, pouvant être liés à des causes diverses et notamment l'induction par des substances pro-inflammatoires potentiellement présentes.

Les épisodes inflammatoires aseptiques sont donc des complications majeures observées après injections de solutions de dialyse.

Si une partie de ces épisodes inflammatoires est liée à un problème d'ordre chimique (injection accidentelle de contaminants chimiques ou mauvais dosages de certains composés), la majorité des cas est directement associée à la présence de contaminants d'origine microbienne présents dans les solutions servant à la préparation des solutions de dialyse.

Les lipopolysaccharides (LPS) et les peptidoglycanes (PGN) sont les principaux contaminants d'origine microbienne présentant un risque élevé de déclencher une inflammation même lorsqu'ils sont présents à l'état de traces.

Il est par ailleurs du mérite de la société Demanderesse d'avoir également pris en compte la présence de molécules susceptibles d'exacerber la réponse inflammatoire induite par ces contaminants, tels que les produits de dépolymérisation des PGN, dont la structure minimale encore bioactive est le muramyl-dipeptide (MDP).

En plus des produits de dépolymérisation des PGN, des peptides microbiens formylés, dont le prototype est le f-MLP (tripeptide formyl-Met-Leu-Phe), ont également une activité synergique importante. A l'origine, ces peptides ont été identifiés pour leur activité chimio-attractante sur les leucocytes, alors qu'ils sont incapables d'induire une réponse cytokinique *per se.*

Il est donc important de ne pas négliger ces "petites molécules" car elles peuvent rendre compte indirectement des épisodes inflammatoires aseptiques en exacerbant les effets de traces de PGN et/ou de LPS.

### Définition de méthodes d'identification et de dosage efficaces desdits contaminants

La société Demanderesse s'est donc employée à développer des méthodes de détection et de dosage plus efficaces que ceux accessibles dans l'état de l'art.

Ces dernières années, de nombreux tests utilisant des cellules primaires se sont développés pour remplacer les modèles animaux dans les tests de réponse inflammatoire.

Toutefois, ces modèles *in vitro* sont sujets à une importante variabilité interindividuelle, ce qui peut être responsable de biais expérimentaux.

A l'inverse, les lignées cellulaires monocytaires donnent des réponses constantes, ce qui explique pourquoi les tests actuellement en développement utilisent de plus en plus ce type de cellules en culture. Cependant, ces tests présentent l'inconvénient de donner une réponse inflammatoire globale à tous les contaminants présents en mélange dans une solution, et par conséquent ne permettent pas de caractériser la nature du contaminant.

Il est également important de noter que la réponse inflammatoire exacerbée est visible pour les cytokines de la phase aiguë de l'inflammation, telles que :
- TNF-α (Tumor Necrosis Factor alpha),
- IL-1β (interleukine 1β) et
- les chimiokines telles que CCL5 (Chemokine (C-C motif) ligand 5) /RANTES (Regulated upon Activation, Normal T-cell Expressed, and Secreted), mais pas ou peu pour l'IL-6 (interleukine 6).

Ainsi, les méthodes basées sur la production de l'IL-6 (US 2009/0239819 et US 2007/0184496) ne sont pas adaptées pour détecter des contaminants en mélange dans une solution.

Il a donc été du mérite de la société Demanderesse d'avoir développé dans sa demande de brevet internationale WO 2012/143647 des méthodes sensibles et efficaces de détection des contaminants microbiens ayant une action pro-inflammatoire, en deçà du seuil de sensibilité des procédures actuellement utilisées et/ou décrites dans la littérature, et ultérieurement d'avoir identifié la famille, voire la nature, des molécules pro-inflammatoires présentes sous formes de traces dans les lots provenant des circuits de production.

### Détermination de l'efficacité des étapes unitaires de purification

La société Demanderesse a ensuite cherché à mieux définir les étapes clefs de purification à mettre en oeuvre sur les polymères de glucose destinés à la dialyse péritonéale.

Pour cela, elle s'est employée à valider les étapes clefs unitaires de purification, en employant les méthodes de détection et dosages basées sur les lignées monocytaires telles que présentées dans sa demande de brevet internationale WO 2012/143647.

Ainsi, dans sa demande de brevet internationale WO 2013/178931, la société Demanderesse a analysé l'efficacité des étapes unitaires suivantes réalisées sur les polymères de glucose destinés à la dialyse péritonéale:
- traitement thermique,
- acidification,
- passage sur charbon actif,
- passage sur des résines d'adsorption, d'ultrafiltration, de filtration,
- hydrolyse chimique ou enzymatique.

Dans une récente demande de brevet non encore examinée, la société Demanderesse s'est ensuite attachée à définir une combinaison de plusieurs étapes de décontamination judicieusement sélectionnées et ordonnées pour leur efficacité à éliminer toutes les molécules inflammatoires susceptibles d'être présentes dans les polymères de glucose issus du procédé de fabrication, quelle que soit la nature de la contamination. Le procédé de cette invention se rapporte ainsi à la combinaison d'étapes suivante, menées sur les polymères de glucose destinés à la dialyse péritonéale :
- traitement par une préparation enzymatique à propriétés détergentes et de clarification,
- traitement par un charbon actif de très haute capacité d'adsorption, de qualité pharmaceutique, et de porosité « micropore » ;
- facultativement, traitement par un second charbon actif de porosité « mésopore »,
- passage sur une résine adsorbante polymérique macroporeuse, présentant une porosité supérieure à 100 Angström ; et
- ultrafiltration en continu sur 5 kDa.

Si ces travaux ont permis de définir la meilleure combinaison apte à sécuriser les polymères de glucose destinés à la dialyse péritonéale de tous contaminants potentiels, il demeure encore un besoin non satisfait d'élaborer en amont un procédé optimisé de décontamination de la source même des contaminants potentiels des circuits de production des polymères de glucose destinés à la dialyse péritonéale, en l'occurrence l'amidon de maïs Waxy (dit cireux) et l'hydrolysat brut d'amidon, qui rentrent dans le circuit de préparation desdits polymères de glucose.

En effet, la mise à disposition d'un procédé susceptible de traiter efficacement l'amidon de maïs Waxy contaminé naturellement par des débris cellulaires de microorganismes de type levures, moisissures ou bactéries, permettrait alors de simplifier tout traitement ultérieur des polymères de glucose qui en sont issus.

Un tel traitement de décontamination à la source sur des produits bruts va conduire effectivement à diminuer la charge en contaminants susceptibles de polluer les circuits de production des polymères de glucose et va contribuer ainsi à les sécuriser, permettant de satisfaire les prérequis des industries pharmaceutiques en matière de degré purification des produits destinés à la dialyse péritonéale.

### Description détaillée de l'invention

La présente invention propose donc une combinaison de plusieurs étapes de décontamination judicieusement sélectionnées et ordonnées qui se révèle efficace pour éliminer toutes les molécules inflammatoires susceptibles d'être présentes dans les amidons et hydrolysats d'amidon utilisés comme matière première pour la préparation de polymères de glucose destinés à la dialyse péritonéale, quelle que soit la nature de la contamination.

Le procédé conforme à l'invention de décontamination des amidons utilisés comme matière première pour la préparation de polymères de glucose destinés à la dialyse péritonéale, le procédé comprenant les étapes suivantes :
- préparation d'un amidon de maïs Waxy,
- mise en suspension de l'amidon Waxy à une concentration comprise entre 20 et 40 % de matière sèche dans une eau de process à un pH compris entre environ 5 et environ 6, en particulier environ 5,5,
- traitement de la suspension d'amidon par une solution d'acide peracétique à une concentration comprise entre 100 et 500 ppm, de préférence de 300 ppm,
- essorage de l'amidon puis reprise dans une eau déminéralisée ajustée à pH compris entre environ 5 et environ 6, en particulier environ 5,5 et à une concentration comprise entre 20 et 40 % de matière sèche,
- élévation de la température comprise entre environ 100°C et 110 °C, de préférence à environ 107°C, puis ajout de l'α-amylase pendant environ 10 à 20 minutes, de préférence environ 15 min,
- facultativement, traitement par une préparation enzymatique à propriétés détergentes et de clarification,
- filtration de la suspension sur lit de diatomées,
- traitement par un charbon actif de très haute capacité d'adsorption, de qualité pharmaceutique, et de porosité « micropore »,
- traitement par un second charbon actif de porosité « mésopore »,
- facultativement, passage sur une résine adsorbante polymérique macroporeuse, présentant une porosité supérieure à 100 Angström,
- facultativement, ultrafiltration en continu sur 5 kDa,
- filtration de sécurité sur filtre stérile de porosité 0,22 µm.
Les étapes du procédé sont à réaliser dans l'ordre d'apparition.
Dans un mode de réalisation préféré, toutes les étapes du procédé, y compris les facultatives, sont réalisés.

Au sens de l'invention, on entend par :
- "eau de process", tout ou partie de l'eau mise en oeuvre dans le circuit d'amidonnerie humide qui y est recyclée (notamment cf. schéma 5 du document *Bilan énérgétique des industries de transformation des céréales dans la CEE* sous internet à l'adresse file:///J:/CDNA10994FRC_001.pdf)
- « préparation enzymatique à propriétés détergentes et de clarification » : des activités enzymatiques de type mannanases, telle la Mannaway® commercialisé par la société Novozymes ;
- « charbon actif de très haute capacité d'adsorption, de qualité pharmaceutique, et de porosité « micropore » : un charbon actif de porosité équivalente au charbon actif Norit C Extra USP ;
- « charbon actif de porosité « mésopore » : un charbon actif de porosité équivalente au charbon actif ENO-PC ;
- « résine adsorbante polymérique macroporeuse, présentant une porosité supérieure à 100 Angström » : une résine de type DOWEX OPTIDORE SD2.
- « environ » signifie plus ou moins 10 % de la valeur, de préférence plus ou moins 5 %. Par exemple, environ 100 signifie entre 90 et 110, de préférence entre 95 et 105. Cela désigne également la valeur exacte.

Les contaminants pro-inflammatoires sont surtout des molécules d'origine bactérienne.

Ils peuvent être en particulier :
- des PGN,
- des LPS,
- des lipopeptides,
- des produits de dépolymérisation des PGN, notamment du MDP,
- les peptides microbiens formylés comme le f-MLP,
- des β glucanes,
- etc...

Les méthodes de mesure des réponses inflammatoires *in vitro* qui sont utilisées dans le cadre de la présente invention pour suivre l'efficacité des étapes de décontamination des procédés de préparation de polymères de glucose à usage thérapeutique chez l'Homme (e.g. solutions de dialyse péritonéale) sont basées sur les tests cellulaires (« *bio-assays* ») utilisant des lignées de type monocytes/macrophages (THP-1, et/ou Raw-Blue™) et des lignées transfectées exprimant un récepteur spécifique de l'immunité naturelle (HEK-Blue™), tests cellulaires développés par la société Demanderesse à partir de lignées cellulaires commerciales et détaillés dans ses demandes de brevet antérieures.

Cinq lignées sont ainsi de préférence utilisées :
- Lignée Raw-Blue™ : cette lignée issue de macrophages murins répond à la majorité des contaminants pro-inflammatoires susceptibles d'être présents dans les matrices et dérivés de polymères de glucose (PGN, lipopeptides, LPS, zymosan, LTA). Son utilisation permet donc d'estimer la charge globale en molécules pro-inflammatoires présentes dans les échantillons.
- lignée HEK-Blue™ hTLR2 : cette lignée exprimant le récepteur hTLR2 répond spécifiquement aux agonistes du TLR2 (PGN et lipopeptides surtout). Son utilisation permet donc de connaître le taux de ces contaminants dans le déclenchement des réponses inflammatoires,

- lignée HEK-Blue™ hTLR4 : cette lignée exprimant le récepteur hTLR4 répond spécifiquement aux LPS. Son utilisation permet donc de connaître le taux de ces contaminants dans le déclenchement des réponses inflammatoires,
- lignée HEK-Blue™ hNOD2 : cette lignée exprimant le récepteur hNOD2 répond spécifiquement aux agonistes de NOD2. Son utilisation permet donc de connaître le taux de MDP et molécules apparentées dans le déclenchement des réponses inflammatoires,
- lignée HEK-Blue™ Null2: il s'agit d'une lignée contrôle, non transfectée par un récepteur de l'immunité. Son utilisation est nécessaire pour vérifier que les solutions de polymères de glucose ou de leurs hydrolysats n'induisent pas la production de la SEAP par un mécanisme de toxicité.

Cependant, il est à noter que l'homme du métier peut également utiliser d'autres lignées commerciales (IMGENEX) ou il peut en préparer.

Dans un mode de réalisation préféré, les lignées cellulaires sont mises en oeuvre à une densité comprise entre 0,5 à 1 x 10⁶ cellules/mL de milieu de culture, et la mise en contact de la préparation de polymères de glucose ou leurs hydrolysats avec les cellules dure environ 16 à 24 h .

Une quantification des contaminants peut être réalisée à l'aide d'une courbe dose-réponse. Cette courbe dose-réponse peut notamment être réalisée avec les mêmes cellules, dans les mêmes conditions, avec des doses croissantes de contaminants. Les courbes doses-réponses sont en particulier réalisées avec des standards de LPS, PGN, lipopeptide et MDP.

De préférence, une telle courbe dose-réponse peut être réalisée pour les cellules exprimant TLR4 (par exemple, THP-1, HEK-Blue™ hTLR4 et Raw-Blue™) avec des doses croissantes de LPS, pour les cellules exprimant TLR2 (par exemple, THP-1, HEK-Blue™ hTLR2 et Raw-Blue™) avec des doses croissantes de PGN, et pour les cellules réactives via NOD2 (par exemple, HEK-Blue™ hNOD2) avec des doses croissantes de MDP.

Les tests cellulaires peuvent être réalisés comme décrit dans les demandes de brevet de la Demanderesse : WO2012/143647 et WO2013/178931.

Après la préparation de la suspension d'amidon waxy à une concentration comprise entre 20 et 40 % de matière sèche dans une eau de process à un pH compris entre environ 5 et environ 6, en particulier environ 5,5, la première étape de décontamination proprement dite du procédé conforme à l'invention consiste en un traitement par l'acide peracétique. L'acide peracétique peut être utilisé à une concentration comprise entre 100 et 500 ppm. De manière préférentielle, on prendra une eau traitée à 300 ppm en acide peracétique. Le temps de contact est d'environ 2 heures, à une température comprise entre environ 5 et environ 15°C, de préférence environ 10°C. Ce traitement de l'amidon par l'acide peracétique a révélé une efficacité pour réduire les contaminants à toute étape ultérieure du procédé, notamment par rapport aux contaminants de type PGN. Cet effet est d'autant plus important que de l'eau de process est utilisée pour préparer la suspension d'amidon.

L'étape suivante, après essorage de l'amidon puis reprise de celui-ci dans une eau déminéralisée ajustée à pH compris entre environ 5 et environ 6, en particulier environ 5,5 et à une concentration comprise entre 20 et 40 % de matière sèche, est la liquéfaction de l'amidon. La liquéfaction de l'amidon est réalisée en plaçant la suspension à une température comprise entre environ 100°C et 110 °C, de préférence à environ 107°C, et en ajoutant l'α-amylase. L'hydrolyse enzymatique dure pendant environ 10 à 20 minutes, de préférence environ 15 min.

L'étape suivante peut consister, de manière optionnelle, en un traitement par une préparation enzymatique à propriétés détergentes et de clarification.

Cette activité enzymatique de type mannanases, telle la préparation enzymatique Mannaway® commercialisée par la société Novozymes qui s'est révélée efficace pour dissocier les macro-complexes tels que des débris bactériens et des PGN de poids moléculaire élevé.

Elle sera donc mise en oeuvre si les analyses de l'amidon liquéfié et hydrolysé révèlent un taux élevé de contaminants de type PGN.

L'activité de cette préparation enzymatique est optimale lorsqu'elle est utilisée à une concentration finale de 0,4 % (vol/vol) dans la solution de polymères de glucose à 32 % (poids/vol) ajustée à pH 10 avec NaOH, pendant un temps de traitement de 24 h à 50°C.

Après traitement, la solution est filtrée sur lit de diatomées, comme il sera exemplifié ci-après.

L'étape suivante consiste ensuite en un traitement par deux charbons actifs en cascade :
1) un premier charbon actif de très haute capacité d'adsorption, de qualité pharmaceutique, de porosité « micropore ».
   La société Demanderesse recommande d'utiliser un charbon actif de type Norit C Extra USP. Le charbon C-extra-USP se montre en effet efficace pour éliminer les PGN et leurs produits de dégradation.
   Son action est maximale lorsqu'il est ajouté à la concentration finale de 0,5% (poids/volume) dans la solution de polymères de glucose à 32 % (poids/vol), ajustée à pH 4,5 avec HCl.
   Le traitement est réalisé sous agitation pendant 1 h à 80°C.
2) un second charbon actif de porosité « mésopore ».
   Ici, un charbon actif de type ENO-PC est préféré. Cette qualité de charbon actif a un spectre d'action large et permet d'éliminer préférentiellement les molécules de poids moléculaire < 100 kDa (par exemple, LPS et produits de dégradation des PGN).
   Il est également utilisé ici à une teneur de 0,5% à pH 4,5 pendant 1 h à une température de 80°C.

La solution obtenue après passage sur ces deux charbons actifs est finalement filtrée sur membrane de seuil de porosité 3 µm.

L'étape suivante optionnelle, consiste en un traitement sur une résine adsorbante polymérique macroporeuse, présentant une porosité supérieure à 100 Angström.

Il est choisi la résine la Dowex SD2, qui présente un spectre plus large d'élimination des molécules contaminantes (autres que les PGN) que d'autres résines de même famille.

Comme il sera exemplifié ci-après, les solutions de polymères de glucose à 32 % (250 mL) sont éluées sur une colonne contenant 20 mL de cette résine.

Cette étape est recommandée pour les matières premières fortement chargées en LPS et/ou traitées par une préparation enzymatique à propriétés détergentes et de clarification.

De manière également optionnelle, l'étape suivante consiste en une ultrafiltration en continu sur une membrane ayant un seuil de coupure à 5 kDa.

Cette étape est recommandée pour les matières premières fortement chargées en produits de dépolymérisation des PGN.

La dernière étape consiste en une filtration de sécurité sur membrane présentant un seuil de coupure de 0,22 µm.

L'invention sera mieux comprise à l'aide des exemples qui suivent, lesquels se veulent illustratifs et non limitatifs.

### Exemples

### Exemple 1 : Caractéristiques des lignées cellulaires utilisées pour les tests de réponses inflammatoires

Les courbes doses-réponses sont réalisées avec des molécules agonistes standards : LPS, PGN et MDP, solubilisées dans une solution de maltodextrine non contaminée (PGN < 1 ng/g ; LPS < 0,5 ng/g; MDP < 0,2 ng/g) à 32 % (poids/volume) dans de l'eau apyrogène (p.p.i.), selon l'enseignement de la demande de brevet internationale WO 2013/178931 de la société Demanderesse.

Les cellules Raw-Blue™ et HEK-Blue™ hTLR2, hTLR4, hNOD2 et Null2 sont incubées avec des concentrations croissantes en agonistes, et la réponse cellulaire est mesurée par quantification de l'activité SEAP :
- Lignée Raw-Blue™ : les cellules répondent aux molécules inflammatoires majeures susceptibles d'être présentes dans les matrices et dérivés de polymères de glucose (PGN et LPS) ; elles ont notamment une forte réactivité vis à vis des PGN, mais ne répondent pas à ses produits de dépolymérisation (MDP).
- Lignée HEK-Blue™ hTLR2 : forte réactivité vis-à-vis des PGN ; les cellules ne montrent aucune réactivité vis-à-vis des LPS et du MDP,
- Lignée HEK-Blue™ hTLR4: forte réactivité vis-à-vis des LPS ; les cellules ne montrent aucune réactivité vis-à-vis des PGN et du MDP,
- Lignée HEK-Blue™ hNOD2: forte réactivité vis-à-vis du MDP ; les cellules ne montrent aucune réactivité vis-à-vis des PGN et du LPS,
- Lignée HEK-Blue™ Null2: témoin d'absence de toxicité cellulaire ; les cellules ne montrent aucune réactivité vis-à-vis des PGN, LPS et MDP.

### Exemple 2 : Préparation des matières premières de polymères de glucose

Les étapes de préparation des matières premières ont toutes été réalisées à l'échelle pilote.

Les matières premières sont préparées à partir d'amidon Waxy mis en suspension à une concentration comprise entre 20 et 40 % de matière sèche (poids/volume).

L'amidon en suspension est laissé une nuit à 4°C, essoré, puis remis en suspension dans de l'eau ajustée à pH 5,5 à une concentration comprise entre 20 et 40 %. La suspension est alors chauffée à 107°C puis traitée en présence d'α-amylase pendant 15 min. Après liquéfaction, la réaction enzymatique est stoppée par addition d'HCl 1N (pH 4), et les produits de liquéfaction sont filtrés sur un lit de diatomées (40 µm).

Selon les essais, l'amidon est mis en solution dans de l'eau déminéralisée ou de l'eau de process, de façon à estimer la part de contamination apportée dans les matières premières par cette eau d'usage courant.

Afin de réduire la charge en contaminants en début de process, la suspension d'amidon peut être traitée par une solution d'acide peracétique à 0,03 %. Dans ce cas, l'amidon Waxy est mis en suspension à une concentration comprise entre 20 et 40 % (p/v), laissé une nuit à 4°C, essoré, remis en suspension puis traité en présence d'acide peracétique (300 ppm). Après un nouvel essorage, l'amidon est remis en suspension dans de l'eau déminéralisée ajusté à pH 5,5 à une concentration comprise entre 20 et 40 % (p/v). Comme précédemment, la solution est chauffée, puis l'α-amylase est ajoutée pour 15 min. La réaction est arrêtée par ajout d'HCl 1N (pH 4) puis filtrée.

Les enseignements de la demande de brevet internationale WO 2013/178931 de la société Demanderesse ont montré que la préparation enzymatique Mannaway® est efficace pour dissocier des macro-complexes tels que des débris bactériens et des PGN de haut poids moléculaire dans une préparation de polymères de glucose finale. Son activité est optimale lorsqu'elle est utilisée à une concentration finale de 0,4 % (vol/vol) dans une solution de polymères de glucose à 32 % (poids/vol) ajustée à pH 8 avec NaOH, pendant un temps de traitement de 24 h à 50°C Après traitement, la solution est neutralisée par HCl et l'enzyme est inactivée par chauffage à 85°C pendant 10 min. Toutefois, la préparation enzymatique Mannaway® est contaminée par des traces de LPS. En outre, des traces d'enzyme peuvent subsister après traitement. De façon à prendre en compte ces contaminations exogènes, l'étape de traitement enzymatique est placée en fin de préparation des matières premières avant la filtration et, par conséquent, avant le début de la procédure de décontamination.

Après chaque étape, des prélèvements sont réalisés pour analyser la charge inflammatoire globale (test avec les cellules Raw-Blue™) et les quantités de contaminants de type PGN, LPS et MDP (réponses des cellules HEK-Blue™).

### Exemple 3 : Comparaison des réponses inflammatoires induites par les matières premières avant décontamination.

L'objectif de ces essais est de déterminer la réactivité pro-inflammatoire des matières premières, d'identifier la nature des bio-contaminants, et de tester les moyens permettant de diminuer leurs avant la mise en oeuvre de la procédure de décontamination. La présence de bio-contaminants dans les différentes matières premières est analysée à l'aide des cinq types cellulaires, de façon à avoir un aperçu des réponses inflammatoires spécifiques à certains contaminants :
- Lignée Raw-Blue™ : tous contaminants avec réactivité élevée pour les PGN,
- Lignée HEK-Blue™hTLR2 : réactivité élevée pour les PGN et les lipopeptides,
- Lignée HEK-Blue™hTLR4 : réactivité élevée pour les LPS,
- Lignée HEK-Blue™hNOD2 : MDP et produits de dépolymérisation des PGN,
- Lignée HEK-Blue™ Null2: témoin d'absence de toxicité cellulaire.

Pour ces tests cellulaires, les matières premières sont diluées dans le milieu de culture des cellules pour obtenir une concentration finale égale à 3,2 % (p/v). Les résultats sont exprimés en activité (réponse SEAP) par rapport à la réponse maximale des cellules.

Essai 1 : L'amidon Waxy a été repris à 20 % dans l'eau à pH 5,5, puis traité en présence d'α-amylase. Deux préparations sont testées :
- préparation 1: amidon Waxy + eau déminéralisée (WD)
- préparation 2 : amidon Waxy + eau de process (WR)

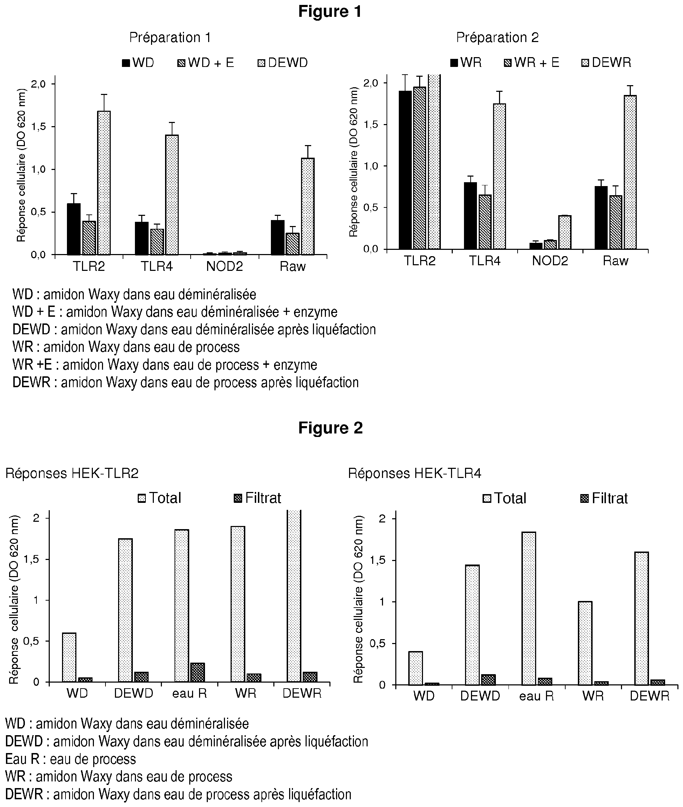

Des prélèvements ont été effectués après chaque étape du process: suspension de l'amidon dans l'eau, addition de l'α-amylase (E), liquéfaction (DE). Les résultats des tests cellulaires sont présentés en Figure 1.

La suspension de l'amidon dans l'eau déminéralisée (WD) a libéré de faibles quantités de PGN et de LPS dans le surnageant (réponses modérées dans les cellules HEK-TLR2, HEK-TLR4 et Raw). L'addition de l'enzyme (WD + E) n'a pas apporté de contamination. Par contre, la liquéfaction a libéré d'importantes quantités de contaminants, comme en témoignent les fortes réponses de l'échantillon DEWD mesurées avec les HEK-TLR2 et HEK-TLR4. Ces résultats indiquent que des PGN et des LPS sont associés aux grains d'amidon et que la liquéfaction a provoqué leur libération.

Par comparaison avec l'eau déminéralisée, l'eau de process contient de fortes quantités de PGN, puisque les réponses TLR2 sont saturées. On observe une augmentation moindre pour la réponse des cellules HEK-TRL4, ce qui indique la présence de LPS dans cette eau de process, mais à des taux plus faibles que les PGN.

Les réponses des cellules HEK-NOD2 ne sont pas significatives avec les deux préparations, ou relativement faible pour celle observée après liquéfaction de l'amidon repris dans l'eau de process. Cette observation suggère que les PGN sont peu dégradés, et donc essentiellement présents sous forme de complexes de taille importante.

Pour vérifier cette hypothèse, les prélèvements ont été filtrés sur des unités de micro-filtration de type Centricon 30 (seuil de coupure 30 kDa). Les tests cellulaires avec HEK-TLR2 et HEK-TLR4 ont été réalisés sur les filtrats et les réponses ont été comparées à celles obtenues avec les produits non filtrés. Les résultats sont présentés en Figure 2.

L'échantillon provenant de la suspension de l'amidon dans de l'eau de process (WR) contient des quantités élevées de PGN et de LPS, qui ne sont pas retrouvées dans les filtrats correspondants. L'eau de process seule (eau R) a également induit de fortes réponses dans les cellules HEK-TLR2 et HEK-TLR4, preuve que les contaminants de type LPS et PGN présents dans le prélèvement WR proviennent en majorité de l'étape de suspension. En outre, les réponses modérées observées avec la suspension dans l'eau déminéralisée (WD) confirment que l'amidon non liquéfié libère peu de contaminants.

A l'inverse, les deux échantillons provenant de la liquéfaction de l'amidon (DEWD et DEWR) sont fortement contaminés. Aucune trace significative de PGN ou de LPS n'est retrouvée dans les filtrats, ce qui confirme que leur présence sous forme de molécules ou d'agrégats de masse moléculaire > 30 kDa. Ces données sont donc en faveur de la présence de grosses molécules, d'agrégats et/ou de débris cellulaires, apportés par l'eau de process et/ou libérés des grains d'amidon par l'étape de liquéfaction.

Essai 2 : Dans cet essai, l'effet de l'acide peracétique a été évalué en réalisant les expériences sur le même lot d'amidon mis en solution dans de l'eau déminéralisée.

L'amidon Waxy a été repris à 20 % dans l'eau à pH 5,5, puis traité en présence d'α-amylase Deux préparations ont été testées :
- préparation 1: amidon Waxy + eau déminéralisée (WD)
- préparation 2 : amidon Waxy + eau déminéralisée puis traitement par l'acide peracétique (WAD).

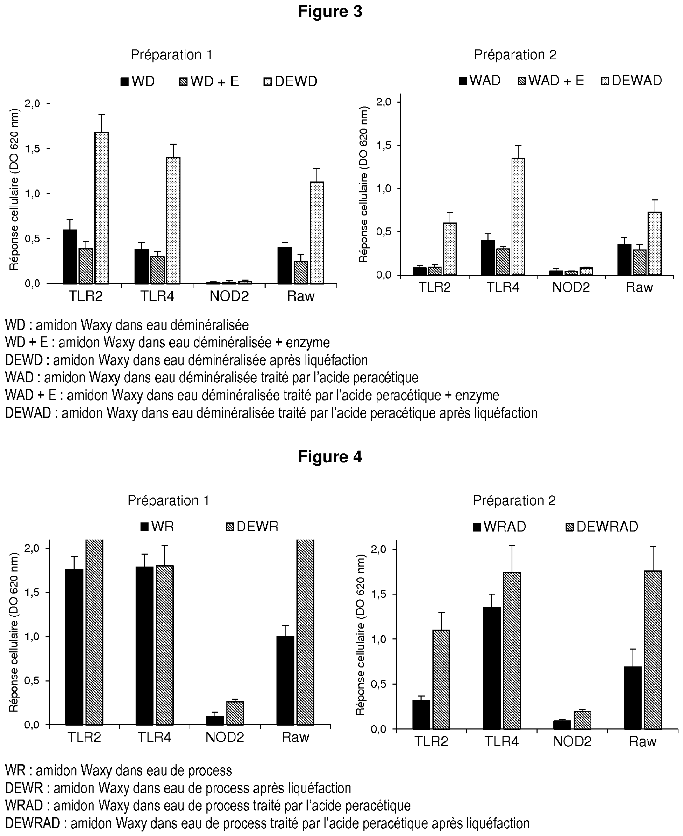

Des prélèvements ont été effectués après chaque étape du process: suspension de l'amidon dans l'eau, addition de l'α-amylase (E), liquéfaction (DE). Les résultats des tests cellulaires sont présentés en Figure 3.

La suspension de l'amidon dans l'eau déminéralisée (préparation 1) a libéré de faibles quantités de PGN et de LPS dans le surnageant (réponses de l'échantillon WD avec HEK-TLR2, HEK-TLR4 et Raw). L'addition de l'enzyme n'a pas apporté de contamination. Par contre, la liquéfaction a libéré de grandes quantités de bio-contaminants, comme en témoignent les fortes réponses obtenues avec l'échantillon DEWD dans les tests HEK-TLR2 et HEK-TLR4. Ces résultats confirment que PGN et LPS sont fortement associés aux grains d'amidon et que la liquéfaction provoque leur solubilisation.

Les résultats obtenus avec la préparation 2 montrent que l'acide peracétique a eu un effet neutralisant sur les PGN associés aux grains d'amidon. En effet, il n'y a pas de réponse TLR2 dans les échantillons avant liquéfaction, puisque les valeurs obtenues avec les échantillons WAD et WAD+E sont au niveau du seuil de détection du dosage. De plus, la réponse TLR2 est nettement réduite après liquéfaction (DEWAD versus DEWD).

En revanche, le traitement a peu d'effet sur les LPS, puisque les réponses TLR4 obtenues avec la préparation 2 sont similaires à celles observées en absence d'acide peracétique pour tous les échantillons. Ces données indiquent que les LPS sont peu sensibles au traitement par l'acide peracétique. De plus, la présence de ces contaminants explique pourquoi l'élimination des PGN n'induit qu'une diminution modérée de la réponse inflammatoire observée avec les cellules Raw.

Les réponses HEK-NOD2 ne sont pas significatives. Cette observation indique que l'action de l'acide peracétique ne s'accompagne pas de la formation de produits de dégradation potentiellement inflammatoires, tels que des petits fragments de PGN et/ou des produits de dépolymérisation de type MDP, mais bien d'une neutralisation de l'activité inflammatoire des PGN.

Essai 3 : Dans cet essai, l'effet de l'acide peracétique a été évalué en réalisant les expériences sur le même lot d'amidon mis en solution dans l'eau de process.

L'amidon Waxy a été repris à environ 30% de matière sèche dans de l'eau à pH 5,5, puis traité en présence d'α-amylase sur un jet cooker. Deux préparations ont été testées :
- préparation 1: amidon Waxy mis en solution dans de l'eau de process, laissé une nuit à 4°C, essoré, puis repris dans de l'eau deau de process ajustée à pH 5,5 (WR).
- préparation 2 : amidon Waxy mis en solution dans de l'eau de process, laissé une nuit à 4°C, puis traité par de l'acide péracétque à 300 ppm. Nouvel essorage puis reprise dans de l'eau déminéralisée ajustée à pH 5,5 (WRAD).

La préparation 1 correspond donc au protocole standard. Dans la préparation 2, l'amidon est repris dans de l'eau déminéralisée après traitement par l'acide peracétique pour ne pas apporter de nouveaux contaminants.

Des prélèvements ont été effectués après les étapes de suspension de l'amidon dans l'eau de process et de liquéfaction. Les résultats des tests cellulaires sont présentés en Figure 4.

Comme attendu, l'eau de process a apporté une quantité importante de PGN solubles dans la préparation 1 (réponse TLR2 pour l'échantillon WR). Après liquéfaction, la réponse TLR2 est saturée (DEWR), ce qui confirme la libération de contaminants de type PGN associés aux grains d'amidon. Par comparaison, le traitement à l'acide peracétique a été très efficace pour réduire la charge en PGN dans la préparation 2, qu'elle soit apportée par l'eau (WRAD) ou libérée par liquéfaction (DEWRAD).

L'eau de process a également apporté une grande quantité de LPS solubles (réponse TLR4 dans l'échantillon WR), mais contrairement à ce qui est observé avec les PGN, la liquéfaction a libéré moins ce type de contaminant (réponses TLR4 pour les échantillons WR et DEWR). Par comparaison, l'acide peracétique a eu un effet modéré sur la charge en LPS, puisqu'on observe une légère diminution de la charge en contaminant apportée par l'eau de process dans la préparation 2 (WRAD).

Dans les deux préparations, l'eau n'est pas chargée en MDP ou fragments de PGN, et la liquéfaction en a libéré une faible quantité (réponses NOD2 similaires pour DEWR et DEWRAD).

Finalement, les réponses des cellules Raw reflétant la charge inflammatoire globale sont réduites dans les échantillons après action de l'acide peracétique, ce qui est en accord avec la perte importante de PGN dans la préparation 2 (WRAD versus WR et DEWRAD versus DEWR). La réactivité résiduelle des cellules Raw après action de l'acide peracétique est donc majoritairement imputable aux LPS apportés par l'eau de process.

Dans l'ensemble, ces données démontrent l'efficacité du traitement par l'acide peracétique pour réduire considérablement la charge en PGN dans la matière première avant la procédure de décontamination. L'avantage de la décontamination se poursuit dans les étapes ultérieures du procédé.

Essai 4 : Les premiers essais suggèrent que la majorité des molécules inflammatoires apportés par l'eau de process et/ou libérés des grains d'amidon par l'étape de liquéfaction sont présents sous forme de complexes de poids moléculaires élevés, tels que des agrégats et/ou des débris cellulaires.

Dans ce nouvel essai, un traitement par l'enzyme Mannaway® a été ajouté entre les étapes de liquéfaction et de filtration, en raison de l'efficacité de cette préparation enzymatique pour dissocier les agrégats et les PGN de haut poids moléculaire.

L'amidon Waxy a été repris à environ 30% dans de l'eau de process à pH 5,5, laissé une nuit, puis traité par de l'acide peracétique à 300 ppm. Après essorage puis reprise dans de l'eau déminéralisée ajustée à pH 5,5, l'α-amylase a été ajoutée pour l'étape de liquéfaction (DEWRAD). La solution a ensuite été ajustée à pH 8 puis traitée en présence de la préparation enzymatique Mannaway® (0,4 %) pendant 24 h à 50°C. Finalement, la solution a été filtrée sur lit de diatomées (DEWRADM).

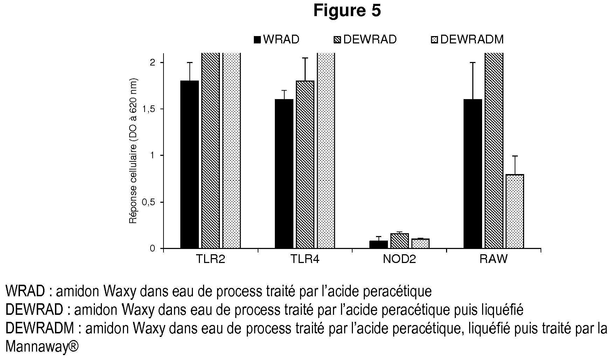

Des prélèvements ont été effectués après les étapes de traitement par l'acide peracétique (WRAD), de liquéfaction (DEWRAD) et d'action de la préparation enzymatique Mannaway® (DEWRADM). Les résultats des tests cellulaires sont présentés en Figure 5.

Comme attendu, l'eau de process a apporté une quantité importante de PGN solubles dans la préparation (WRAD). Dans cet essai, l'eau devait être particulièrement contaminée en PGN, puisque les réponses TLR2 et Raw sont encore très élevées après action de l'acide peracétique (WRAD), et largement saturées après liquéfaction (DEWRAD). Toutefois, on peut noter une diminution de la réponse Raw après action de la Mannaway®, ce qui est la preuve que la préparation enzymatique a bien éliminé une partie des contaminants inflammatoires (DEWRADM versus DEWRAD).

Contrairement au PGN, la charge en LPS est peu modifiée après liquéfaction, preuve que la majeure partie est apportée par l'eau de process. Par contre, après addition de la Mannaway®, on observe une forte augmentation de la réponse TLR4, ce qui était prévisible étant donné que cette solution est elle-même contaminée par des LPS.

Ce dernier résultat indique qu'il faudra certainement tenir de cet apport exogène de LPS lors de la procédure de décontamination.

### Exemple 4 : Effet des procédures de décontamination sur les réponses inflammatoires induites par les matières premières.

Différents traitements de décontamination des polymères de glucose (sous forme de produit fini) ont déjà été testés individuellement et en combinaison, et rapportés dans la demande de brevet internationale WO 2013/178931 de la société Demanderesse. Ces travaux ont permis d'identifier les traitements les mieux adaptés à chaque type de contaminants présents dans les échantillons et de déterminer les conditions pour leur application sur des matrices de polymères de glucose.

Les inventeurs ont voulu tester l'efficacité de ces traitements, mis au point sur un produit fini à l'étape de purification ultime, sur un mélange complexe tel que l'hydrolysat d'amidon.

Les traitements retenus sont :
- traitement sur charbons actifs : les charbons actifs retenus pour la présente étude sont : C-extra-USP, pour son efficacité à éliminer les PGN ; ENO-PC, pour son spectre large sur les contaminants de poids moléculaire < 100 kDa (par exemple, LPS et produits de dégradation des PGN).
   L'action des charbons est maximale lorsqu'ils sont ajoutés à la concentration finale de 0,5% (poids/volume) dans la solution de polymères de glucose à 32 % (poids/vol), ajustée à pH 4,5 avec HCl 1N. Le traitement est réalisé sous agitation pendant 1 h à 80°C. Après traitement, la solution (500 mL) est neutralisée par NaOH puis filtrée sur fritté (porosité 3 µm). Etant donné que les traitements par charbons sont réalisés en *batch* et nécessitent des étapes de chauffage, neutralisation et filtration, ils sont réalisés avant les autres traitements.
- passage sur résines d'adsorption : les résines retenues pour la présente étude sont : Dowex SD2, pour son spectre large d'élimination des contaminants ; MN-100, pour son efficacité à retenir les molécules de type LPS.

Pour les expériences, les solutions de polymères de glucose à 32 % (250 mL) sont éluées sur une colonne contenant 20 mL de chaque résine. Les enseignements de l'étude précédente ont montré que cette procédure ne provoque pas de phénomène de saturation des résines par les solutions de polymères de glucose.
- ultrafiltration sur 5 kDa : le traitement par ultrafiltration a pour objectif d'éliminer les molécules de petite taille (produits de dégradation des PGN et MDP) encore présentes dans les solutions de polymères de glucose. Cette étape est donc facultative et utilisée en fin de procédure si la réponse NOD2 est positive.

Les essais sont réalisés en injectant en continu la solution de polymères de glucose sur un filtre 5 kDa à un débit de 25 mL/min pendant 3 h à température ambiante. Pour compenser la perte du filtrat, le rétentat est injecté dans la solution de départ et ajusté en continu au volume initial (100 mL) par addition d'eau déminéralisée stérile.

Les différentes étapes de décontamination ont été réalisées en laboratoire. Après chaque étape, des prélèvements ont été réalisés en condition stérile et utilisés dans les tests cellulaires, afin de doser la charge inflammatoire globale (réponse Raw) et les quantités de bio-contaminants (réponses TLR2, TLR4 et NOD2). Pour les réponses cellulaires saturées, les échantillons ont été préalablement dilués (1/10^{ème} et 1/100^{ème}).

Les concentrations en contaminants ont été calculées en se reportant aux courbes doses-réponses réalisées avec des molécules agonistes standards : LPS, PGN et MDP, décrites dans l'exemple 1 et établies selon l'enseignement de la demande de brevet internationale WO 2013/178931 de la société Demanderesse.

Les concentrations en contaminants ont ensuite été ramenées à la quantité de polymère de glucose présente dans l'échantillon. Puis, les valeurs obtenues après chaque étape de décontamination ont été comparées à celle de la matière première de départ, de façon à estimer l'efficacité des procédures de décontamination. Les résultats sont exprimés en pourcentage d'abattement par rapport à la charge initiale en contaminants et en contamination résiduelle par rapport aux seuils limites de détection (SLD) de chaque *bio-assay* (en ng par g de polymère de glucose) : HEK-TLR2, < 1 ng PGN ; HEK-TLR4, < 0,5 ng LPS ; HEK-NOD2, < 0,2 ng MDP ; Raw, < 2 ng PGN.

Les procédures suivantes ont été analysées :
- Procédure 1 : Dans ce premier essai de décontamination, la matière première a été préparée en suivant le protocole décrit dans l'exemple 3, essai 3 : amidon Waxy (à environ 20 %) mis en solution dans l'eau de process, laissé une nuit à 4°C (WR), puis traité par de l'acide peracétique à 300 ppm. Essorage puis reprise dans l'eau déminéralisée à pH 5,5 (WRAD). Liquéfaction puis filtration sur lit de diatomées (DEWRAD).

La matière première correspondant à l'échantillon DEWRAD a ensuite été décontaminée en utilisant la combinaison suivante :
1. traitement par charbon C extra USP (0,5%), suivi d'une filtration sur fritté (3 µm),
2. traitement par charbon ENO-PC (0,5%), suivi d'une filtration sur fritté (3 µm),
3. passage sur colonne de résine SD2,
4. filtration sur filtre stérile (0,22µm).

Des prélèvements ont été effectués après les différentes étapes de préparation de la matière première puis de la procédure de décontamination.

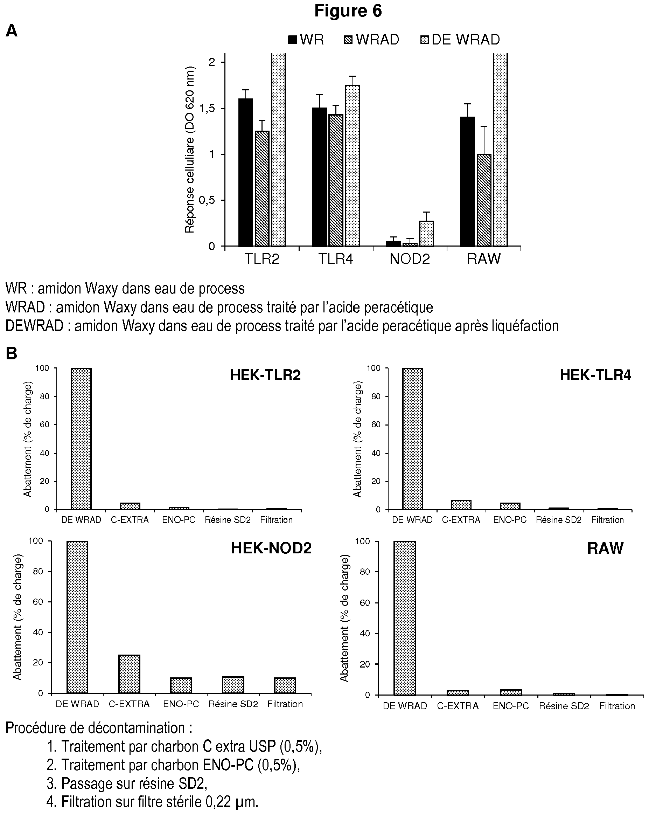

Les résultats des tests cellulaires sont présentés en Figure 6.

Tout d'abord, le traitement par l'acide peracétique (WRAD versus WR) a réduit la teneur en PGN avant liquéfaction (réponses TLR2 et Raw), mais n'a pas eu d'effet significatif sur les LPS (réponse TLR4). La liquéfaction a libéré des quantités importantes de PGN associés aux grains d'amidon, puisque les réponses TLR2 et Raw sont saturées pour l'échantillon DEWRAD, alors que la réponse TLR4 n'est que très légèrement augmentée. On peut en conclure que dans cet essai, l'amidon était très chargé en PGN, car on a une saturation des réponses TLR2 et Raw, même après l'action de l'acide peracétique (Figure 6A).

Pour calculer l'efficacité des étapes de décontamination, les concentrations initiales en contaminants ont été déterminées à partir de l'échantillon DEWRAD pour chaque type cellulaire ; pour les réponses cellulaires saturées (TLR2 et Raw), l'échantillon a été préalablement dilué au 1/100^{ème}, puis les valeurs de concentration ont été corrigées par le facteur de dilution. Les concentrations résiduelles en contaminants ont été calculées après chaque étape de décontamination, puis les valeurs ont été rapportées aux concentrations initiales de façon à exprimer les résultats en pourcentage d'abattement (Figure 6B et Tableau I).

De manière tout à fait surprenante, la procédure de décontamination a permis une diminution très marquée de la réponse TLR2, avec un abattement de la charge en PGN > 99,9 % (seuil de détection). En outre, les charbons C-extra-USP et ENO-PC en série ont un effet additif sur l'élimination des PGN avant passage sur résine, ce qui conforte le choix de ces deux charbons pour leur action complémentaire.

La procédure est également efficace pour réduire la réponse NOD2, étant donné que le seuil limite de détection est atteint pour cette lignée cellulaire en fin de process. Comparativement au PGN, l'abattement n'est que de 90 %, mais cette valeur est liée au fait que les agonistes NOD2 (produits de dépolymérisation des PGN et MDP) étaient présents à l'état de traces dans la matière première de départ.

L'élimination des LPS est > 99,9 % en fin de procédure, et la réponse TLR4 atteint également le seuil limite de détection. On peut remarquer que le passage sur la résine SD2 est pour atteindre ce seuil de décontamination. En effet, des traces significatives de LPS sont encore présentes après traitement par les deux charbons. Toutefois, la matière était fortement contaminée en LPS, ce qui peut expliquer pourquoi l'action combinée des deux charbons n'a pas été suffisante pour tout éliminer.

Finalement, la réponse des cellules Raw confirme l'efficacité de cette première procédure pour éliminer tous les types de contaminants présents dans la matière première. En effet, on n'observe plus de réponse inflammatoire significative (< seuil limite de détection), ce qui reflète un abattement > 99,9 % de la charge inflammatoire globale.

| Tableau I : Valeurs d'abattement (en % de la charge initiale) et de charge résiduelle en fin de process de décontamination | | | | |
|---|---|---|---|---|
| | HEK-TLR2 | HEK-TLR4 | HEK-NOD2 | Raw |
| Abattement (%) | > 99,9 | > 99,9 | > 90 | > 99,9 |
| Charges résiduelles | < 1 ng/g | < 0,5 ng/g | < 0,2 ng/g | < SLD |

| | | | | |
|---|---|---|---|---|
| *SLD, seuil limite de détection* | | | | |

- Procédure 2 : le premier essai suggère que la résine SD2 peut être utilisée de manière facultative à condition que le taux de LPS ne soit pas trop élevé dans la matière première. Pour tester cette hypothèse, une matière première a été préparée en suivant le protocole décrit précédemment : amidon Waxy (à environ 20 %) mis en solution dans l'eau de process, laissé une nuit à 4°C (WR), puis traité par de l'acide peracétique à 300 ppm. Essorage puis reprise dans l'eau déminéralisée à pH 5,5 (WRAD). Liquéfaction puis filtration sur lit de diatomées (DEWRAD).

La matière première correspondant à l'échantillon DEWRAD a ensuite été décontaminée en utilisant une combinaison « simplifiée » sans passage sur résine SD2.
1. traitement par charbon C extra USP (0,5%) suivi d'une filtration sur fritté (3 µm),
2. traitement par charbon ENO-PC (0,5%) suivi d'une filtration sur fritté (3 µm),
3. filtration sur filtre stérile (0,22µm).

Des prélèvements ont été effectués après les différentes étapes de préparation de la matière première puis de la procédure de décontamination.

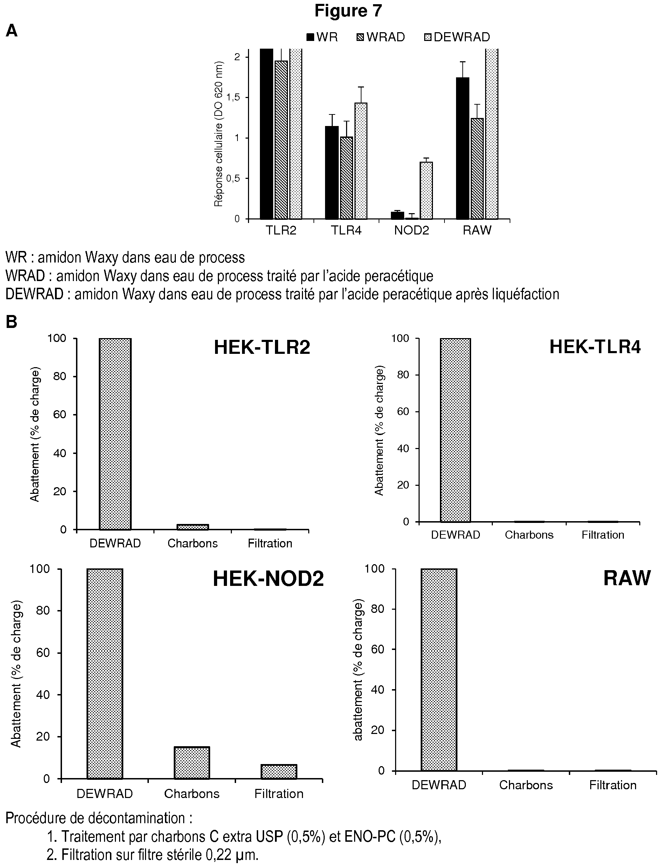

Les résultats des tests cellulaires sont présentés en Figure 7.

Avant liquéfaction, la réponse TLR2 obtenue avec l'échantillon WR est saturée, ce qui indique que l'eau de process est très contaminée par des PGN. Le traitement par l'acide peracétique réduit partiellement cette contamination (WRAD), mais la liquéfaction libère de nouveaux PGN, puisque la réponse TLR2 est à nouveau saturée avec l'échantillon DEWRAD. A l'inverse, les contaminations en LPS (réponses TLR4) restent modérées, qu'elles proviennent de l'eau de process ou de la liquéfaction. La matière première utilisée pour cette nouvelle procédure est donc plus fortement contaminée en PGN que la précédente, mais moins en LPS (Figure 7A).

L'échantillon DEWRAD a ensuite été traité selon la procédure de décontamination associant les deux charbons C-Extra et ENO-PC et la filtration sur filtre 0,22 µm. Les charges initiales en contaminants contenues dans l'échantillon DEWRAD ont été ramenées à 100 % et les pourcentages d'abattement relatifs ont été calculés à partir des charges résiduelles en contaminants après chaque étape (Figure 7B).

La procédure de décontamination a permis un abattement très marquée de la réponse TLR2 (> 99,9 %), et ce, malgré la forte contamination en PGN dans la matière première et l'absence de passage sur résine SD2. Ce résultat confirme l'efficacité des charbons pour éliminer ce type de contaminant. La combinaison est également suffisante pour réduire la charge en produits de dépolymérisation des PGN, étant donné que le seuil limite de détection de la réponse NOD2 est atteint en fin de process.

Contrairement au premier essai de décontamination, la résine SD2 n'apparait pas non plus nécessaire ici pour réduire la contamination en LPS. En effet, la réponse TLR4 atteint également le seuil limite de détection, et l'élimination des LPS est > 99,9 % en fin de procédure.

Finalement, la réponse des cellules Raw confirme l'efficacité de cette procédure « simplifiée » pour éliminer les contaminants présents dans une matière première peu chargée en LPS. En effet, on n'observe plus de réponse inflammatoire significative en fin de procédure (< seuil limite de détection).
- Procédure 3 : dans ce troisième essai de décontamination, la matière première a été préparée en suivant le protocole décrit dans l'exemple 3, essai 4, dans lequel un traitement par l'enzyme Mannaway® a été ajouté entre les étapes de liquéfaction et de filtration sur lit de diatomées. En effet, la préparation enzymatique a prouvé son efficacité pour dissocier les agrégats et les PGN de haut poids moléculaire.

Par contre, la Mannaway® est contaminé par du LPS. De façon à éliminer cet apport exogène, la résine SD2 a été remplacée par la résine MN-100, pour son efficacité à retenir les molécules de type LPS lors des essais unitaires.

Préparation : amidon Waxy (à environ 30% de MS) mis en solution dans de l'eau de process, laissé une nuit à 4°C, puis traité par de l'acide peracétique à 0,03%. Essorage puis reprise dans de l'eau déminéralisée ajustée à pH 5,5 (WRAD). Addition de l'α-amylase et liquéfaction (DEWRAD). Ajustement à pH 8 et traitement par la Mannaway® (0,4 %) pendant 24 h à 50°C (DEWRADM).

La matière première correspondant à l'échantillon DEWRADM a ensuite été décontaminée en utilisant la combinaison suivante :
1. traitement par charbon C extra USP (0,5%) suivi d'une filtration sur fritté (3 µm),
2. traitement par charbon ENO-PC (0,5%) suivi d'une filtration sur fritté (3 µm),
3. passage sur colonne de résine MN-100,
4. filtration sur filtre stérile (0,22 µm).

Des prélèvements ont été effectués après les différentes étapes de préparation de la matière première puis de la procédure de décontamination.

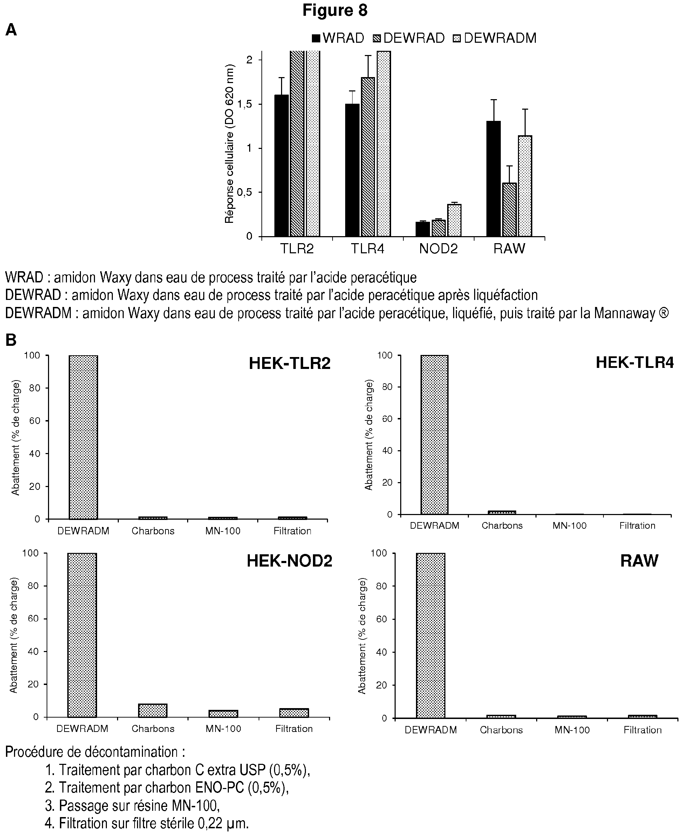

Les résultats des tests cellulaires sont présentés en Figure 8.

Avant liquéfaction, la réponse TLR2 obtenue avec l'échantillon WRAD n'est pas saturée, ce qui indique que le traitement par l'acide peracétique a été efficace pour réduire la contamination en PGN apporté par l'eau de process. Par contre, la liquéfaction libère de nouveaux PGN, puisque la réponse TLR2 est saturée avec l'échantillon DEWRAD. La contamination en LPS est élevée dans l'eau de process (WRAD), et comme attendu, la liquéfaction ne modifie pas significativement la réponse TLR4. Par contre, l'addition de la Mannaway® apporte une forte contamination en LPS exogène, puisque la réponse TLR4 induite par l'échantillon DEWRADM est saturée. La matière première utilisée pour ce nouvel essai est donc très fortement contaminée en PGN et en LPS (Figure 8A).

L'échantillon DEWRADM a ensuite été traité selon la procédure associant les deux charbons C-Extra et ENO-PC, la résine MN-100 et la filtration sur filtre 0,22 µm. Les charges initiales en contaminants contenues dans l'échantillon DEWRADM ont été ramenées à 100 % et les pourcentages d'abattement relatifs ont été calculés à partir des charges résiduelles après chaque étape (Figure 8B et Tableau II).

La procédure de décontamination a été très efficace pour éliminer les LPS, puisqu'on observe un abattement marquée de la réponse TLR4 (> 99,8 %), qui atteint le seuil limite de détection du dosage. La résine MN-100 a donc retenu ces contaminants, qu'ils soient apportés par l'eau de process ou par la préparation enzymatique Mannaway®.

La combinaison est également efficace pour éliminer les produits de dépolymérisation des PGN, étant donné que le seuil limite de détection de la réponse NOD2 est également atteint en fin de procédure.

Par contre, il demeure une réponse TLR2 (équivalente à 5,2 ng de PGN par g de matière sèche), malgré un abattement d'environ 99 % de la charge inflammatoire en fin de process. Ces données indiquent que des agonistes TLR2 sont encore présents à l'état de traces dans la matière première, et ceci malgré l'efficacité combinée des deux charbons pour éliminer les PGN.

La réponse des cellules Raw confirme la présence de contaminants inflammatoires dans la matière première en fin de procédure. En effet, l'abattement de la charge inflammatoire globale n'est que de 98,5 %, et la réponse est significativement au-dessus du seuil limite de détection (équivalente à 8 ng de PGN par g de matière sèche).

| Tableau II : Valeurs d'abattement (en % de la charge initiale) et de charge résiduelle en fin de process de décontamination | | | | |
|---|---|---|---|---|
| | HEK-TLR2 | HEK-TLR4 | HEK-NOD2 | Raw |
| Abattement (%) | 98,9 | > 99,8 | > 90 | 98,4 |
| Charges résiduelles | 5,2 ng/g (PGN) | < 0,5 ng/g | < 0,2 ng/g | 8 ng/g (PGN) |

Les essais précédents ayant montré l'efficacité des deux charbons pour éliminer les PGN, même dans des matières premières très contaminées, ces résultats suggèrent que la préparation enzymatique Mannaway® a apporté des agonistes TLR2 de nature chimique différente des PGN. Ces contaminants peuvent être par exemple des lipopeptides, connus pour être de forts inducteurs des réponses TLR2. Ainsi, l'association charbons + résine MN-100, bien qu'efficace pour retenir les PGN et les LPS, aurait laissé passer ce type de contaminant, ce qui expliquerait les réponses TLR2 et Raw résiduelles.

Pour pallier ce problème, la résine MN-100 a été remplacée par la résine SD2 dans l'essai suivant, en raison de son spectre d'action plus large.
- Procédure 4 : dans cet essai, la matière première correspond à l'échantillon DEWRADM utilisée pour la procédure 3. La procédure de décontamination reprend les étapes précédentes, mais en remplaçant la résine MN-100 par la résine SD2 à large spectre :
   1. traitement par charbon C extra USP (0,5%) suivi d'une filtration sur fritté (3 µm),
   2. traitement par charbon ENO-PC (0,5%) suivi d'une filtration sur fritté (3 µm),
   3. passage sur colonne de résine SD2,
   4. filtration sur filtre stérile (0,22 µm).

Des prélèvements ont été effectués après les différentes étapes de préparation de la matière première puis de la procédure de décontamination.

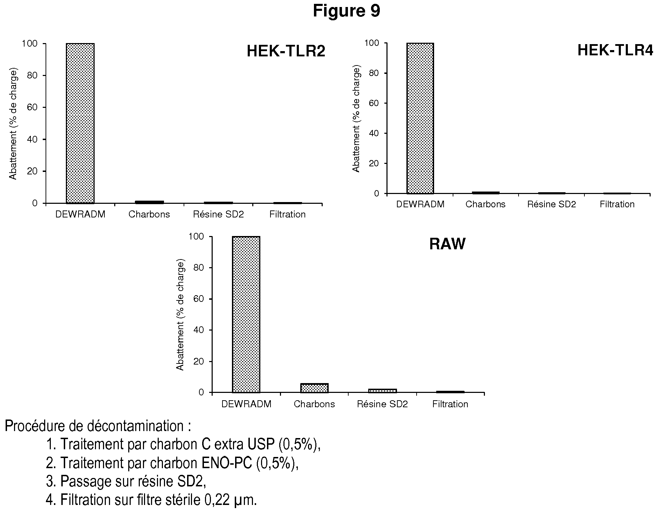

Les résultats des tests cellulaires sont présentés en Figure 9.

Les abattements relatifs pour chaque contaminant ont été calculés à partir des charges résiduelles mesurées après chaque étape de la combinaison et sont exprimés en pourcentages par rapport aux charges initiales contenues dans l'échantillon DEWRADM (Figure 9 et Tableau III).

Contrairement à la combinaison testée en procédure 3, cette combinaison reprenant la résine SD2 permet d'éteindre la réponse des cellules HEK-TLR2 au niveau du seuil limite de détection, avec un abattement de la charge inflammatoire > 99,9 %. Ainsi, l'association charbons C-extra-USP et ENO-PC en série et résine SD2 a apparemment un effet complémentaire sur l'élimination des agonistes TLR2, qu'ils soient de type PGN ou autre. La procédure garde également son efficacité pour éliminer les contaminants agonistes de NOD.

Le choix de la résine MN-100 était basé sur la faite que la préparation enzymatique Mannaway® est contaminée par des LPS. Le remplacement par la résine SD2 permet une élimination des LPS tout aussi efficace, avec un abattement > 99,9 % en fin de procédure (seuil limite de détection). On peut donc conclure de ses résultats que la résine SD2 est au final une meilleure option que la résine MN-100 pour éliminer les LPS et les autres contaminants apportés par la Mannaway® dans la matière première.

Finalement, la réponse des cellules Raw confirme l'efficacité de cette procédure pour éliminer tous les types de contaminants présents dans la matière première. En effet, on n'observe plus de réponse inflammatoire significative (< seuil limite de détection), ce qui reflète un abattement > 99,9 % de la charge inflammatoire globale.

| Tableau III : Valeurs d'abattement (en % de la charge initiale) et de charge résiduelle en fin de process de décontamination | | | | |
|---|---|---|---|---|
| | HEK-TLR2 | HEK-TLR4 | HEK-NOD2 | Raw |
| Abattement (%) | > 99,9 | > 99,9 | > 90 | > 99,9 |
| Charges résiduelles | < 1 ng/g | < 0,5 ng/g | < 0,2 ng/g | < SLD |

| | | | | |
|---|---|---|---|---|
| *SLD, seuil limite de détection* | | | | |

### Bilan :

Dans leur ensemble, les résultats obtenus dans cette étude montrent que la combinaison de plusieurs étapes de production et de décontamination judicieusement sélectionnées et ordonnées se révèle efficace pour éliminer les molécules inflammatoires susceptibles d'être présentes dans des matières premières utilisées pour la préparation polymères de glucose.

La combinaison comprend les étapes suivantes :
- mise en suspension de l'amidon Waxy à une concentration comprise entre 20 et 40 % de matière sèche dans une eau de process à un pH = 5,5,
- traitement de la suspension d'amidon par une solution d'acide peracétique à une concentration finale comprise entre 100 et 500 ppm, de préférence de 300 ppm,
- essorage de l'amidon puis reprise dans une eau déminéralisée ajustée à pH 5,5 à une concentration comprise entre 20 et 40 % de matière sèche,
- élévation de la température à 107°C puis ajout de l'α-amylase pendant 15 min,
- facultativement, traitement par une préparation enzymatique à propriétés détergentes et de clarification, par exemple Mannaway®, pour les matières premières fortement chargées en PGN,
- filtration de la suspension sur lit de diatomées,
- traitement par un charbon actif de porosité équivalente à C-Extra-USP,
- traitement par un second charbon actif de porosité équivalente à ENO-PC,
- facultativement, passage sur une résine d'adsorption de type Dowex-SD2, pour les matières premières fortement chargées en LPS et/ou traitées par une préparation enzymatique à propriétés détergentes et de clarification, par exemple Mannaway®,
- facultativement, ultrafiltration en continu sur 5 kDa, pour les matières premières fortement chargées en produits de dépolymérisation des PGN,
- filtration de sécurité sur filtre stérile de proposité 0,22 µm.

La combinaison de ces étapes permet de cibler les différentes familles de contaminants et de proposer des matières premières de polymères de glucose exemptes de réactivité inflammatoire.

### Figures

**Figure 1** **:** Réponses cellulaires induites par les matières premières préparées soit dans de l'eau déminéralisée, soit dans de l'eau de process. Les résultats sont exprimés en valeurs d'absorbance mesurées à 620 nm (test SEAP).
**Figure 2** **:** Réponses cellulaires induites par les matières premières non filtrées et par les filtrats obtenus après ultra-filtration 30 kDa. Les résultats sont exprimés en valeurs d'absorbance mesurées à 620 nm (test SEAP).
**Figure 3** **:** Réponses cellulaires induites par les matières premières préparées dans de l'eau déminéralisée et traitées ou non par de l'acide peracétique. Les résultats sont exprimés en valeurs d'absorbance mesurées à 620 nm (test SEAP).
**Figure 4** **:** Réponses cellulaires induites par les matières premières préparées dans de l'eau de process et traitées ou non par de l'acide peracétique. Les résultats sont exprimés en valeurs d'absorbance mesurées à 620 nm (test SEAP).
**Figure 5** **:** Réponses cellulaires induites par les matières premières préparées dans de l'eau de process et traitées par la Mannaway®. Les résultats sont exprimés en valeurs d'absorbance mesurées à 620 nm (test SEAP).
**Figure 6** **: (A)** Réponses cellulaires induites par la matière première préparée pour la procédure 1 de décontamination. Les résultats sont exprimés en valeurs d'absorbance mesurées à 620 nm (test SEAP). **(B)** Abattements de la charge en contaminants au cours de la procédure 1. Les valeurs de charge sont obtenues à partir des courbes doses-réponses pour chaque type cellulaire et exprimées en pourcentages par rapport à celles obtenues pour l'échantillon DEWRAD ramenées à 100%.
**Figure 7** **: (A)** Réponses cellulaires induites par la matière première préparée pour la procédure 2. Les résultats sont exprimés en valeurs d'absorbance mesurées à 620 nm (test SEAP). **(B)** Abattements de la charge en contaminants au cours de la procédure « simplifiée » de décontamination. Les valeurs de charge sont obtenues à partir des courbes doses-réponses et exprimées en pourcentages par rapport à celles obtenues pour l'échantillon DEWRAD ramenées à 100%.
**Figure 8** **: A)** Réponses cellulaires induites par la matière première préparée pour la procédure 3. Les résultats sont exprimés en valeurs d'absorbance mesurées à 620 nm (test SEAP). **(B)** Abattements de la charge en contaminants au cours de la procédure 3. Les valeurs de charge sont obtenues à partir des courbes doses-réponses et exprimées en pourcentages par rapport à celles obtenues pour l'échantillon DEWRADM ramenées à 100%.
**Figure 9** **:** Abattements de la charge en contaminants au cours de la procédure 4. Les valeurs de charge sont obtenues à partir des courbes doses-réponses et exprimées en pourcentages par rapport à celles obtenues pour l'échantillon DEWRADM ramenées à 100%.

## Revendications

1. Procédé de décontamination des amidons utilisés comme matière première pour la préparation de polymères de glucose destinés à la dialyse péritonéale, le procédé comprenant les étapes suivantes :
- préparation d'un amidon de maïs Waxy,
- mise en suspension de l'amidon Waxy à une concentration comprise entre 20 et 40 % de matière sèche dans une eau de process à un pH compris entre environ 5 et environ 6, en particulier environ 5,5,
- traitement de la suspension d'amidon par une solution d'acide peracétique à une concentration comprise entre 100 et 500 ppm, de préférence de 300 ppm,
- essorage de l'amidon puis reprise dans une eau déminéralisée ajustée à pH compris entre environ 5 et environ 6, en particulier environ 5,5 et à une concentration comprise entre 20 et 40 % de matière sèche,
- élévation de la température comprise entre environ 100°C et 110 °C, de préférence à environ 107°C puis ajout d'une α-amylase pendant environ 10 à 20 minutes, de préférence environ 15 min,
- facultativement, traitement par une préparation enzymatique à propriétés détergentes et de clarification,
- filtration de la suspension sur lit de diatomées,
- traitement par un charbon actif de très haute capacité d'adsorption, de qualité pharmaceutique, et de porosité « micropore »,
- traitement par un second charbon actif de porosité « mésopore »,
- facultativement, passage sur une résine adsorbante polymérique macroporeuse, présentant une porosité supérieure à 100 Angström,
- facultativement, ultrafiltration en continu sur 5 kDa,
- filtration de sécurité sur filtre stérile de porosité 0,22 µm.

2. Procédé selon la revendication 1, **caractérisé en ce que** la préparation enzymatique à propriétés détergentes et de clarification présente une activité enzymatique de type mannanases.

3. Procédé selon la revendication 1 ou 2, **caractérisé en ce que** l'étape de traitement par une préparation enzymatique à propriétés détergentes et de clarification est réalisé si l'amidon liquéfié et hydrolysé révèle un taux élevé de contaminants de type PGN.

4. Procédé selon l'une quelconque des revendications 1 à 3, **caractérisé en ce que** toutes les étapes, y compris les facultatives, sont réalisés.

## Patentansprüche

1. Verfahren zur Dekontaminierung von Stärke als Rohmaterial zur Gewinnung von Glucosepolymeren für die Peritonealdialyse, wobei das Verfahren die folgenden Schritte umfasst:
- Zubereitung einer Wachsmaisstärke,
- Suspendieren der Wachsstärke mit einer Konzentration von 20 bis 40% Trockenmasse in Prozesswasser bei einem pH-Wert von ungefähr 5 bis ungefähr 6, insbesondere von ungefähr 5,5,
- Behandlung der Stärkesuspension mit einer Peressigsäure-Lösung mit einer Konzentration von 100 bis 500 ppm, bevorzugt von 300 ppm,
- Entwässern der Stärke, dann Aufnahme in demineralisiertem Wasser mit einem auf ungefähr 5 bis ungefähr 6 eingestellten pH-Wert, insbesondere ungefähr 5,5, und bei einer Konzentration von 20 bis 40% Trockenmasse,
- Erhöhung der Temperatur von ungefähr 100 bis 110 °C, bevorzugt auf ungefähr 107 °C, dann hinzufügen einer a-Amylase während ungefähr 10 bis 20 Minuten, bevorzugt ungefähr 15 Minuten,
- fakultativ Behandlung mit einer Enzymzubereitung mit reinigenden und klärenden Eigenschaften,
- Filtration der Suspension in einem Diatomeenbett,
- Behandlung mit einer Aktivkohle mit sehr hoher Absorptionskapazität von pharmazeutischer Qualität und mit "Mikroporen"-Porosität,
- Behandlung mit einer zweiten Aktivkohle mit "Mesoporen"-Porosität,
- fakultativ Führen über ein makroporöses absorbierendes Polymerharz mit einer Porosität größer 100 Angström,
- fakultativ kontinuierliche Ultrafiltration bei 5 kDa,
- Sicherheitsfiltration mit sterilem Filter der Porosität 0,22 µm.

2. Verfahren nach Anspruch 1, **dadurch gekennzeichnet, dass** die Enzymzubereitung mit reinigenden und klärenden Eigenschaften eine Enzymaktivität vom Mannanase-Typ aufweist.

3. Verfahren nach Anspruch 1 oder 2, **dadurch gekennzeichnet, dass** der Schritt der Behandlung mit einer Enzymzubereitung mit reinigenden und klärenden Eigenschaften ausgeführt wird, wenn die verflüssigte und hydrolysierte Stärke eine erhöhte Menge an Verunreinigungen vom PGN-Typ aufweist.

4. Verfahren nach einem der Ansprüche 1 bis 3, **dadurch gekennzeichnet, dass** alle Schritte einschließlich der fakultativen Schritte, durchgeführt werden.

## Claims

1. A method for decontaminating the starches used as raw material for the preparation of glucose polymers intended for peritoneal dialysis, the method comprising the following steps:
- preparing a waxy corn starch,
- suspending the waxy starch at a concentration of between 20 and 40% dry matter in a process water at a pH of between approximately 5 and approximately 6, in particular approximately 5.5,
- treating the suspension of starch with a solution of peracetic acid at a concentration of between 100 and 500 ppm, preferably of 300 ppm,
- removing excess water from the starch then taking up in a demineralized water adjusted to a pH of between approximately 5 and approximately 6, in particular approximately 5.5 and at to a concentration of between 20 and 40% of dry matter, to provide a liquefied starch,
- raising the temperature between approximately 100°C and 110°C, preferably to approximately 107°C, then adding an α-amylase for approximately 10 to 20 minutes, preferably approximately 15 minutes,
- optionally treating with an enzymatic preparation with detergent and clarifying properties,
- filtering the suspension over a bed of diatoms,
- treating with a pharmaceutical-grade activated carbon with very high adsorption capacity and "microporous" porosity,
- treating with a second activated carbon with "mesoporous" porosity,
- optionally passing over a macroporous adsorbent polymer resin having a porosity of greater than 100 Angström, and
- optionally, continuous 5 kDa ultrafiltration,
- safety filtration over a sterile filter with a porosity of 0.22 µm.

2. The method as claimed in claim 1, **characterized in that** that the enzymatic preparation with detergent and clarifying properties has enzymatic activity of mannanase type.

3. The method as claimed in claim 1 or 2, **characterized in that** the step of treating with an enzymatic preparation with detergent and clarifying properties is carried out if the liquefied and hydrolyzed starch proves to have has a high level of PGN-type contaminants.

4. The method as claimed in any one of claims 1 to 3, **characterized in that** all the steps, including according the optional steps, are carried out.
